# EUROPÄISCHE PATENTSCHRIFT

(11) **EP 2 016 913 B1**
(45) Veröffentlichungstag und Bekanntmachung des Hinweises auf die Patenterteilung: **03.03.2010**
(21) Anmeldenummer: 07014255.9
(22) Anmeldetag: 20.07.2007
(51) Int. Cl.: A61B 17/16, A61B 17/32

(54) **Endoskopisches Instrument**
Endoscopic instrument
Instrument endoscopique

(43) Veröffentlichungstag der Anmeldung: 21.01.2009
(73) Patentinhaber: Richard Wolf GmbH, 75438 Knittlingen (DE)
(72) Erfinder: Körner, Eberhard Dipl.-Ing, 75438 Knittlingen (DE); Schmid, Franc, 75248 Ölbronn-Dürrn (DE); Prestel, Stephan, 76287 Rheinstetten-Mörsch (DE)
(74) Vertreter: Vollmann, Heiko

(56) Entgegenhaltungen:
- WO-A-03/022162
- DE-A1- 10 036 108
- DE-A1- 10 324 844
- US-A1- 2005 261 692

## Beschreibung

Die Erfindung betrifft ein endoskopisches Instrument zum Abtragen von Material, Gewebe und dergleichen mit den im Oberbegriff des Anspruchs 1 angegebenen Merkmalen.

Aus dem Bereich der Medizin sind Instrumente bekannt, die über natürliche oder künstlich geschaffene Zugangskanäle in Körperhöhlen einführbar sind, um dort mit diesen Instrumenten Gewebe abzutragen. Solche Instrumente weisen üblicherweise einen durch den Zugangskanal in die Körperhöhle einführbaren Hohlschaft auf, an dessen distalen Ende in Verlängerung des Hohlschaftes ein rotierendes Werkzeug zum Abtragen des Gewebes angeordnet ist. Insbesondere bei engen Zugangskanälen stoßen diese Instrumente dann an ihre Grenzen, wenn sich das abzutragende Gewebe seitlich einer durch den Zugangskanal definierten Zugangsachse befindet.

Es sind Instrumente bekannt, bei denen der distale Endbereich mit dem daran befindlichen Werkzeug starr abgewinkelt ausgebildet ist. Solche Instrumente sind beispielsweise in US 1,677,337, US 4,646,738 sowie DE 43 23 756 A1 beschrieben. Diese Instrumente mit einem starr abgewinkelten Hohlschaftende und Werkzeug sind aber für den endoskopischen Einsatz nicht geeignet, da sie nicht durch einen geraden Arbeitskanal eines Endoskops durchgeführt werden können.

Hierfür grundsätzlich geeignete Instrumente sind in US 5,669,926 A, US 5,851,212 A und US 6,464,711 B1 beschrieben. Diese Instrumente weisen einen geraden Hohlschaft auf, der durch den geraden Arbeitskanal eines Endoskops durchführbar ist. Zum Abtragen von seitlich des Hohlschaftes gelegenem Körpergewebe sind die Hohischäfte mit dem daran angeordneten Werkzeug quer zu ihrer ursprünglichen Längsachse abkrümmbar. Allerdings ist der Biegeradius der Hohlschäfte bei diesen instrumenten verhältnismäßig groß, so dass die an dem distalen Ende des Hohlschaftes angeordneten Werkzeuge außerhalb des Erfassungsbereichs einer Endoskopoptik liegen können und ein mit diesen Instrumenten vorgenommener operativer Eingriff in der Regel mit zusätzlichen optischen Mitteln beobachtet werden muss. Ein weiterer Nachteil dieser Instrumente ist darin zu sehen, dass bei Einsatz eines rotierend betriebenen Werkzeugs eine durch den Hohlschaft geführte Antriebswelle ebenfalls biegbar, d. h. flexibel ausgebildet sein muss, wodurch mit diesen Antriebswellen nur verhältnismäßig kleine Drehmomente bei gleichzeitig niedrigen Drehzahlen übertragen werden können. Aus diesem Grund sind diese Instrumente nicht zum Abtragen harter Gewebe wie z. B. Knochengewebe geeignet.

Aus US 2005/0261692 A1 ist ein instrument bekannt, bei dem die Antriebswelle zweigeteilt ausgebildet ist, wobei die beiden starren Antriebswellenteile relativ zueinander abwinkelbar miteinander verbunden sind. Die Antriebswelle ist in einem Hohlschaft geführt. Dieser Hohlschaft ist ebenfalls zweiteilig ausgebildet, wobei ein distales Hohlschaftteil korrespondierend zur Ausgestaltung der Antriebswelle relativ zu einem proximalen Hohlschaftteil abwinkelbar ist.

Vor diesem Hintergrund ist es die Aufgabe der Erfindung, ein endoskopisches Instrument zum Abtragen von Material, Gewebe und dergleichen zu schaffen, das durch einen geraden Arbeitskanal eines Endoskops durchführbar ist, wobei es gleichzeitig zum Abtragen harter Materialien und Gewebe geeignet sein soll, die auch seitlich zu einer Verlängerung des Arbeitskanals des Endoskops angeordnet sein können.

Diese Aufgabe wird erfindungsgemäß durch ein endoskopisches Instrument mit den im Anspruch 1 angegebenen Merkmalen gelöst. Bevorzugte Ausführungsformen ergeben sich aus den Unteransprüchen, der nachfolgenden Beschreibung und der Zeichnung.

Das endoskopische Instrument gemäß der Erfindung weist einen starren Hohlschaft und ein Werkzeug am distalen Instrumentenende auf. Das Werkzeug ist über eine innerhalb des Hohlschaftes geführte Antriebsweile rotierend antreibbar. Erfindungsgemäß ragt das Werkzeug an dem distalen Ende des Hohlschaftes aus dem Hohlschaft heraus, wobei es gegenüber dem distalen Ende des Hohlschaftes abwinkelbar ist.

D. h., das Werkzeug kann von einer ersten Werkzeugstellung, in der es eine gerade Verlängerung des Hohlschaftes bildet, quer zu seiner Längs- bzw. Rotationsachse in zumindest eine zweite Werkzeugstellung abgewinkelt werden. Derart ausgebildet kann das erfindungsgemäße endoskopische Instrument einerseits in der ersten Werkzeugstellung durch den Arbeitskanal eines Endoskops zu seinem Arbeitsbereich innerhalb eines Hohlraums geführt werden, andererseits ist es mit diesem Werkzeug in einer abgewinkelten Werkzeugstellung möglich, Material oder Gewebe in einem Bereich abzutragen, der sich seitlich einer Verlängerung der Längsachse von Hohlschaft bzw. Arbeitskanal des Endoskops befindet. Indem lediglich das Werkzeug und nicht Bereiche des Hohlschaftes abgewinkelt werden, befindet sich das Werkzeug auch in der abgewinkelten Werkzeugstellung im Erfassungsbereich der Optik des Endoskops, so dass das Werkzeug exakt zu bzw. an dem abzutragenden Material oder Gewebe positioniert werden kann.

Über entsprechende Kupplungsmittel ist das Werkzeug vorteilhaft derart mit der Antriebswelle bewegungsgekoppelt, dass es sowohl in der ersten als auch in der zweiten Werkzeugstellung von der Antriebswelle antreibbar ist. Bei dem Werkzeug kann es sich beispielsweise um ein Fräs- oder Bohrwerkzeug oder einen so genannten "Shaver" handeln.

Ein endoskopisches Instrument im Sinne der vorliegenden Erfindung ist auch ein Technoskop für technische Bearbeitungsaufgaben in Form eines Materialabtrags in ansonsten schwer zugänglichen Hohlräumen von Maschinen, Motoren und ähnlichem. Bevorzugt handelt es sich bei dem erfindungsgemäßen Instrument allerdings um ein medizinisch genutztes endoskopisches Instrument, das über den Arbeitskanal eines Endoskops zum Abtragen von Gewebe, insbesondere Knochengewebe in einen menschlichen oder tierischen Körper einführbar ist.

Das Werkzeug ist in einem Kopfteil zur Lagerung des Werkzeugs angeordnet. Dieses Kopfteil ist an dem distalen Ende des Hohlschaftes angelenkt. Außenseitig des distalen Endes des Hohlschaftes bildet das Kopfteil eine Aufnahme für das Werkzeug. In dieser Aufnahme ist das Werkzeug vorzugsweise drehbeweglich gelagert und lösbar befestigt. Das Kopfteil ist im Bereich des distalen Endes des Hohlschaftes bevorzugt um eine quer zur Längsachse des Hohlschaftes ausgerichtete. Achse schwenkbar, wobei das Kopfteil von einer Stellung, in der das in dem Kopfteil gelagerte Werkzeug in Verlängerung der Längsachse des Hohlschaftes angeordnet ist, in eine Stellung bewegbar ist, in der das Werkzeug gegenüber der Längsachse des Hohlschaftes abgewinkelt ist.

Um das Werkzeug auch in dieser gegenüber dem Hohlschaft abgewinkelten Stellung mit der in dem Hohlschaft geführten Antriebswelle wirkungsverbinden zu können, ist vortellhafterweise ein Kardangelenk vorgesehen, das das Werkzeug mittel- oder unmittelbar mit der Antriebswelle bewegungskoppelt. Das Kardangelenk ermöglicht das Abwinkeln des Werkzeugs gegenüber der in dem Hohlschaft geführten Antriebswelle. So können die Antriebswelle und das Werkzeug in einer ersten Stellung achsgleich angeordnet sein, während die Rotationsachse des Werkzeugs in einer zweiten Stellung bezogen auf die Rotationsachse der Antriebswelle abgewinkelt sein kann. In beiden Stellungen gewährleistet das Kardangelenk aufgrund seiner großen Steifigkeit in Rotationsrichtung die Übertragung verhältnismäßig großer Drehmomente auch bei großen Drehzahlen, was insbesondere das Abtragen harter Materialien bzw. Gewebe, insbesondere das Abtragen von Knochengewebe, ermöglicht.

Das Werkzeug kann direkt mit dem Kardangelenk gekoppelt sein. Bevorzugt ist allerdings die Antriebswelle zumindest zweigeteilt, wobei das Kardangelenk zwischen zwei Antriebswellenteilen angeordnet ist. Bei dieser Ausgestaltung verbindet das Kardangelenk ein proximalseitiges mit einem Antriebsmotor wirkungsverbundenes Antriebswellenteil mit einem distalseitigen Antriebswellenteil, das mit dem Werkzeug bewegungsgekoppelt ist.

An dem proximalen Ende des erfindungsgemäßen Instruments ist zweckmäßigerweise ein Griffteil vorgesehen. In diesem Griffteil kann die Antriebswelle mit dem Antriebsmotor bewegungsgekoppelt sein. Um eine den hygienischen Anforderungen entsprechende Reinigung des endoskopischen Instruments gewährleisten zu können, kann die Antriebswelle auch distalseitig des Griffteils und proximalseitig des Kardangelenkteils teilbar ausgebildet sein. Hierbei können ein griffteilseitiges und zumindest ein hohlschaftseitiges Wellenteil vorgesehen sein, wobei diese beiden Antriebswellenteile vorzugsweise derart ausgebildet sind, dass sie miteinander formschlüssig verbindbar sind.

Um das Werkzeug in eine gegenüber dem distalen Ende des Hohlschaftes abgewinkelte Stellung bewegen zu können, ist das Kopfteil zweckmäßigerweise über ein Gelenkteil mit einer in dem Hohlschaft in Axialrichtung bewegbaren Betätigungsstange bewegungsgekoppelt. Das Gelenkteil ist vorzugsweise an dem distalen Ende der Betätigungsstange und an dem Kopfteil angelenkt, wobei es an dem Kopfteil zweckmäßigerweise auch in einer Richtung quer zur Längsachse des Kopfteils verschiebbar ist. Mittels dieser Ausgestaltung ist es vorteilhaft mit nur einer aus der Befestigungsstange und dem Gelenkteil bestehenden Vorrichtung möglich, das Kopfteil mit dem darin angeordneten Werkzeug durch Verschieben der Betätigungsstange in Achsrichtung des Hohlschaftes sowohl in eine zu dem distalen Ende des Hohlschaftes abgewinkelte Stellung als auch zurück in eine mit dem Hohlschaft fluchtende Stellung zu verschwenken.

Zum Verschieben der Betätigungsstange in dem Hohlschaft weist das erfindungsgemäße Instrument vorteilhafterweise proximalseitig eine Handhabe auf, die mit der Betätigungsstange bewegungsgekoppelt ist. Diese Handhabe ist vorzugsweise an einem proximalseitig des Instruments angeordneten Griffteil vorgesehen und mit der Betätigungsstange bewegungsgekoppelt. Bei der Handhabe kann es sich beispielsweise um einen Betätigungshebel oder Betätigungsschieber handeln, mit dem das Kopfteil mit dem darin gelagerten Werkzeug sowohl in eine gegenüber dem Ende des Hohlschaftes abgewinkelte Stellung als auch in eine mit dem distalen Ende des Hohlschaftes fluchtende Stellung bewegbar sind. Bevorzugt ist die Handhabe als Hebel ausgebildet.

Insbesondere dann, wenn das erfindungsgemäße endoskopische Instrument für den medizinischen Bereich vorgesehen ist, kann innerhalb des Hohlschaftes vorteilhafterweise ein Kanal ausgebildet sein, über den in einem Operationsgebiet abgetrennte Gewebepartikel abgesaugt werden können, oder über den eine Spülflüssigkeit zu dem Operationsgebiet zugeführt werden kann. Dieser Kanal mündet zweckmäßigerweise im Bereich des distalen Endes des Hohlschaftes. Sein proximales Ende ist zweckmäßigerweise mit Absaugmitteln bzw. mit Mitteln zum Zuführen einer Spülflüssigkeit leitungsverbunden. In einer bevorzugten Ausgestaltung bildet die innerhalb des Hohlschaftes angeordnete Antriebswelle den Kanal zum Zuführen und/oder Abführen von Spülflüssigkeit. D. h., bei dieser Ausgestaltung ist die Antriebswelle als Hohlwelle ausgebildet.

In einer weiteren bevorzugten Ausgestaltung bildet die Betätigungsstange einen Kanal zum Zuführen und/oder Abführen von Spülflüssigkeit. Dementsprechend ist die Betätigungsstange in diesem Fall bevorzugt als ein Rohr ausgebildet.

Nachfolgend ist die Erfindung anhand eines in der Zeichnung dargestellten Ausführungsbeispiels näher erläutert. Darin zeigen:

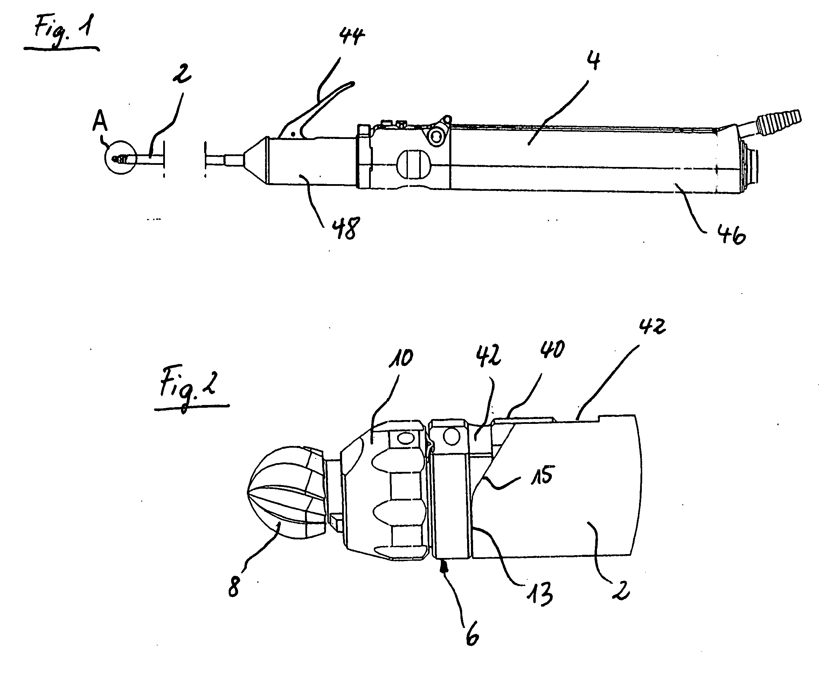

- Fig. 1: in schematischer Darstellung ein endoskopisches Instrument gemäß der Erfindung in einer Seitenansicht,
- Fig. 2: die Einzelheit A aus Fig. 1 in vergrößerter Darstellung,
- Fig.3: das endoskopische Instrument nach Fig. 1 mit einem Werkzeug in abgewinkelter Stellung,
- Fig. 4: die Einzelheit B aus Fig. 3 in vergrößerter Darstellung,
- Fig. 5: einen distalen abkoppelbaren Endbereich des Instruments nach Fig. 1,
- Fig. 6: eine Antriebswelle mit einem daran angeordneten Werkzeug des Instruments nach Fig. 1 und
- Fig. 7: eine teilgeschnittene Seitenansicht des distalen Endbereichs des Instruments nach Fig. 1.

Das in den Figuren dargestellte endoskopische Instrument weist einen starren Hohlschaft 2 auf. Dieser Hohlschaft 2 ist distalseitig eines Griffteils 4 angeordnet. Der Hohlschaft 2 bildet den Teil des endoskopischen Instruments, das über den Arbeitskanal eines nicht dargestellten Endoskops in das Körperinnere eingeführt wird. Außenseitig des distalen Endes des Hohlschaftes 2 ist ein Kopfteil 6 angeordnet, in dem ein Werkzeug in Form eines Fräskopfes 8 gelagert ist. Das Kopfteil 6 weist an seinem distalen Ende ein Verschlusselement 10 auf, mit dem der Fräskopf 8 in dem Kopfteil 6 lösbar befestigt werden kann. Hierzu ist in dem Verschlusselement 10 eine Einspannvorrichtung vorgesehen, mit der der Fräskopf 8 kraftschlüssig festgeklemmt werden kann.

An einem Gelenk 12 am distalen Ende des Hohlschaftes 2 ist das Kopfteil 6 um eine Achse C schwenkbar gelagert. Die Achse C erstreckt sich normal zu einer Längsachse D des Hohlschaftes 2. Die Schwenkbarkeit des Kopfteils 6 ermöglicht es, den Fräskopf 8 gegenüber dem distalen Ende des Hohlschaftes 2 abzuwinkeln. Um das Kopfteil 6 gegenüber dem distalen Ende des Hohlschaftes 2 abwinkeln zu können, ist die distale Stirnseite des Hohlschaftes 2 teilweise abgeschrägt. So ist ein Bereich 13 des distalen Endes des Hohlschaftes 2 normal zur Längsachse D des Hohlschaftes 2 ausgerichtet, während ein sich daran anschließender Bereich 15 hierzu schräg verläuft. Sind das Kopfteil 6 und der darin gelagerte Fräskopf 8 in direkter Verlängerung des Hohlschaftes 2 angeordnet, liegt das proximale Ende des Kopfteils 6 an dem Bereich 13 an. In der abgewinkelten Stellung des Kopfteils 6 kommt es an dem Bereich 15 zur Anlage.

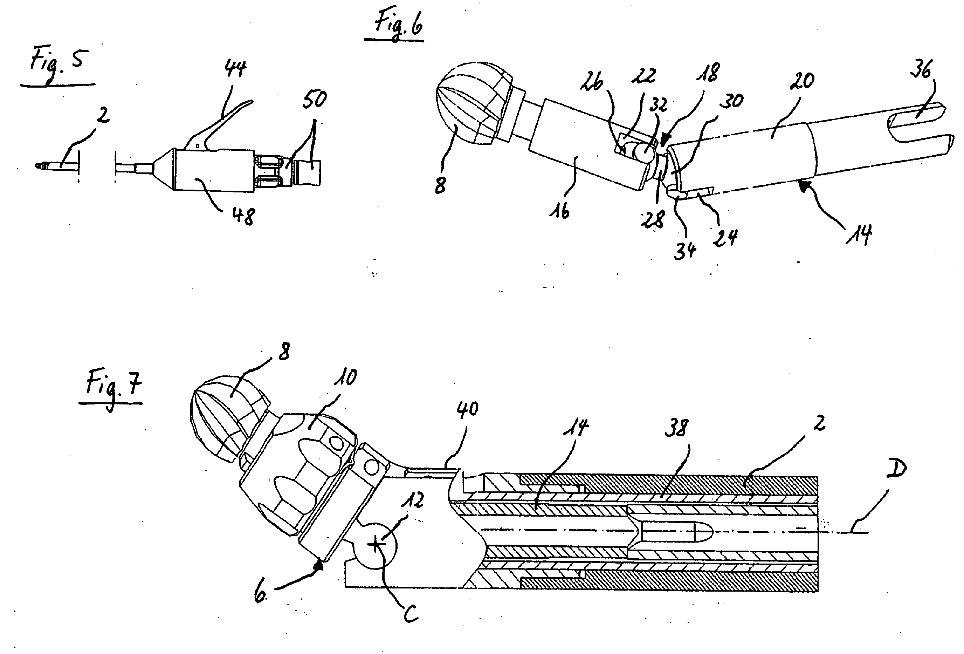

Mittels einer hohlen Antriebswelle 14 (Fig. 6) ist der Fräskopf 8 mit einem in dem Griffteil 4 angeordneten Antriebsmotor wirkungsverbunden. Die Antriebswelle 14 ist mehrteilig ausgebildet, wobei ein distales Antriebswellenteil 16, in das der Fräskopf 8 formschlüssig eingreift, über ein Kardangelenk 18 mit einem sich proximalseitig daran anschließenden Antriebswellenteil 20 verbunden ist, das in Antriebsverbindung mit dem in dem Griffteil 4 angeordneten Antriebsmotor steht.

Zur Bildung des Kardangelenks 18 weist das Antriebswellenteil 16 an seinem proximalen Ende einen senkrecht zur dortigen Stirnfläche verlaufenden Schlitz 22 und das Antriebswellenteil 20 an seinem distalen Ende einen senkrecht zur dortigen Stirnfläche verlaufenden Schlitz 24 auf.

Daneben ist ein hantelförmiges Gelenkteil vorgesehen, bei dem ein Kugelkörper 26 über einen Steg 28 mit einem Kugelkörper 30 verbunden ist. Durch den Kugelkörper 26 ist ein Stift 32 derart geführt, dass die beiden Enden des Stiftes 32 jeweils aus dem Kugelkörper 26 herausragen. Der Stift 32 ist quer zu dem Steg 28, der die Kugelkörper 26 und 30 miteinander verbindet, ausgerichtet. In ähnlicher Weise ist durch den Kugelkörper 30 ein Stift 34 geführt, dessen Enden auch jeweils aus dem Kugelkörper 30 herausragen. Auch der Stift 34 ist quer zu dem Steg 28 ausgerichtet, wobei er allerdings gegenüber dem Stift 32 um im Wesentlichen 90° versetzt ist. Zur Verbindung der Antriebswellenteile 16 und 18 greift der Kugelkörper 26 in das proximale Ende des hohlen Antriebswellenteils 16 ein, wobei die Enden des Stifts 32 in dem Schlitz 22 des Antriebswellenteils 16 geführt sind. In ähnlicher Weise greift der Kugelkörper 30 in das distale Ende des ebenfalls hohlen Antriebswellenteils 20 ein, wobei die Enden des Stifts 34 in dem an dem Antriebswellenteil 20 ausgebildeten Schlitz 24 geführt sind.

An seinem proximalen Ende weist das Antriebswellenteil 20 einen quer über die gesamte Stirnfläche verlaufenden Schlitz 36 auf. Dieser Schlitz dient zur Kupplung mit einem weiteren griffseitigen nicht dargestellten Antriebswellenteil, das an seinem distalen Ende einen mit dem Schlitz 36 korrespondierenden Vorsprung aufweist, der zur Kupplung dieses Antriebswellenteils mit dem Antriebswellenteil 20 in den Schlitz 36 formschlüssig in Form einer Klauenkupplung eingreift.

Zum Abwinkeln des Kopfteils 6 mit dem darin gelagerten Fräskopf 8 ist eine Betätigungsstange 38 vorgesehen. Diese Betätigungsstange 38 ist innerhalb des Hohlschaftes 2 zwischen der Antriebswelle 14 und der Innenwandung des Hohlschaftes 2 angeordnet. An ihrem distalen Ende weist die Betätigungsstange 38 ein Endstück 40 auf, das sich gegenüber der Betätigungsstange 38 in radialer Richtung des Hohlschaftes nach außen hin erweitert und in einem an dem distalen Ende des Hohlschaftes 2 vorgesehenen Längsschlitz 42 geführt ist. An dem Endstück 40 der Betätigungsstange 38 ist ein Gelenkteil 42 angelenkt. Beabstandet von der Anlenkung an dem Endstück 40 der Betätigungsstange 38 ist das Gelenkteil 42 auch an dem Kopfteil 6 angelenkt.

Die Betätigungsstange 38 ist in dem Hohlschaft 2 in Richtung der Längsachse D verschiebbar geführt. Betätigt wird die Betätigungsstange 38 mittels einer an dem Griffteil 4 angeordneten Handhabe in Form eines Hebels 44. Durch Bewegung der Betätigungsstange 38 in proximaler Richtung wird das Kopfteil 6 in seine abgewinkelte Stellung verbracht, in der es an dem Bereich 15 der distalen Stirnseite des Hohlschaftes 2 anliegt. Wird die Betätigungsstange 38 in distaler Richtung bewegt, schwenkt das Kopfteil 6 wieder in eine solche Stellung, in der es in direkter Verlängerung des Hohlschaftes 2 angeordnet ist.

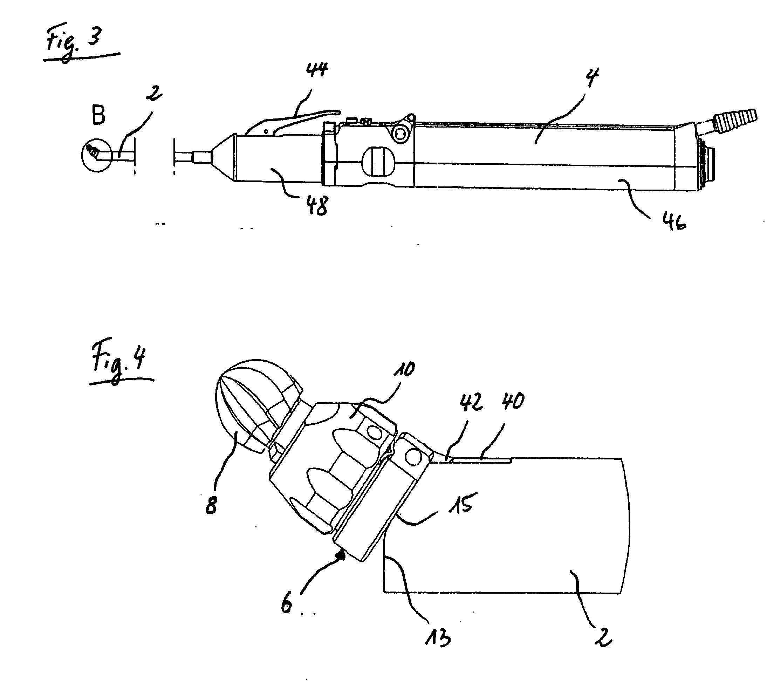

Das Griffteil 4 des endoskopischen Instruments ist teilbar ausgebildet und weist ein proximalseitiges Griffteil 46 und ein distalseitiges Griffteil 48 (Fig. 3) auf. In dem Griffteil 46 des Instruments ist dessen Antriebsmotor angeordnet. An das Griffteil 48 schließt sich distalseitig der Hohlschaft 2 mit dem Kopfteil 6 und dem Fräskopf 8 an. Darüber hinaus ist an dem Griffteil 48 der Hebel 44 zum Betätigen der Betätigungsstange 38 angeordnet. Zum lösbaren Verbinden der Griffteile 46 und 48 sowie zur lösbaren Kupplung der Antriebswelle 14 mit dem Antriebsmotor sind an dem Griffteil 48 Kupplungsmittel 50 vorgesehen.

### Bezugszeichen

- 2: Hohlschaft
- 4: Griffteil
- 6: Kopfteil
- 8: Fräskopf
- 10: Verschlusselement
- 12: Gelenk
- 13: Bereich
- 14: Antriebswelle
- 15: Bereich
- 16: Antriebswellenteil
- 18: Kardangelenk
- 20: Antriebswellenteil
- 22: Schlitz
- 24: Schlitz
- 26: Kugelkörper
- 28: Steg
- 30: Kugelkörper
- 32: Stift
- 34: Stift
- 36: Schlitz
- 38: Betätigungsstange
- 40: Endstück
- 42: Gelenkteil
- 44: Hebel
- 46: Griffteil
- 48: Griffteil
- 50: Kupplungsmittel
- A: Einzelheit
- B: Einzelheit
- C: Achse
- D: Längsachse

## Patentansprüche

1. Endoskopisches Instrument zum Abtragen von Material, Gewebe und dergleichen mit einem starren Hohlschaft (2) und mit einem Werkzeug (8) am distalen Instrumentenende welches über eine in dem Hohlschaft (2) geführte Antriebswelle (14) rotierend antreibbar ist, wobei das an dem distalen Ende des Hohlschafts (2) herausragende Werkzeug gegenüber dem distalen Ende des Hohlschaftes (2) abwinkelbar ist, wobei das Werkzeug (8) in einem an dem distalen Ende des Hohlschafts (2) abwinkelbar angelenkten Kopfteil (6) zur Lagerung des Werkzeugs (8) angeordnet ist, **dadurch gekennzeichnet, dass** das Werkzeug (8) mittels eines Verschlusselements (10) in dem Kopfteil (6) lösbar befestigt ist.

2. Endoskopisches Instrument nach Anspruch 1, **dadurch gekennzeichnet, dass** das Werkzeug (8) über ein Kardangelenk (18) mittel- oder unmittelbar mit der Antriebswelle (14) bewegungskoppelt ist.

3. Endoskopisches Instrument nach einem der vorangehenden Ansprüche, **dadurch gekennzeichnet, dass** die Antriebswelle (14) zumindest zweiteilig ausgebildet ist.

4. Endoskopisches Instrument nach einem der vorangehenden Ansprüche, **dadurch gekennzeichnet, dass** das Kopfteil (6) über ein Gelenkteil (42) mit einer in dem Hohlschaft (2) in Axialrichtung bewegbaren Betätigungsstange (38) bewegungsgekoppelt ist.

5. Endoskopisches Instrument nach Anspruch 4, **dadurch gekennzeichnet, dass** das Instrument proximalseitig eine Handhabe (44) aufweist, die mit der Betätigungsstange (38) bewegungsgekoppelt ist.

6. Endoskopisches Instrument nach einem der vorangehenden Ansprüche, **dadurch gekennzeichnet, dass** innerhalb des Hohlschafts (2) zumindest ein Kanal ausgebildet ist, der im Bereich des distalen Hohlschaftendes mündet.

7. Endoskopisches Instrument nach einem der vorangehenden Ansprüche, **dadurch gekennzeichnet, dass** die Antriebswelle einen Kanai zum Zuführen und/oder Abführen von Spülflüssigkeit bildet.

8. Endoskopisches Instrument nach einem der Ansprüche 4 bis 5, **dadurch gekennzeichnet, dass** die Betätigungsstange (38) einen Kanal zum Zuführen und/oder Abführen von Spülflüssigkeit bildet.

## Claims

1. An endoscopic instrument for the removal of material, tissue or likewise, with a rigid hollow shank (2) and with a tool (8) at the distal instrument end, said tool being driven in rotation via a drive shaft (14) led in the hollow shank (2), wherein the tool projecting out at the distal end of the hollow shank (2) may be bent with respect to the distal end of the hollow shank (2), wherein the tool (8) is arranged in a head part (6) for mounting the tool (8), said head part being articulated in a bendable manner at the distal end of the hollow shank (2), **characterised in that** the tool (8) is releasably fastened in the head part (6) by way of a closure element (10).

2. An endoscopic instrument according to claim 1, **characterised in that** the tool (8) is coupled in movement in a direct or indirect manner to the drive shaft (14) via a universal joint (18).

3. An endoscopic instrument according to one of the preceding claims, **characterised in that** the drive shaft (14) is designed of at least two parts.

4. An endoscopic instrument according to one of the preceding claims, **characterised in that** the head part (6) via a joint part (42) is coupled in movement to an actuation rod (38) movable in the hollow shank (2) in the axial direction.

5. An endoscopic instrument according to claim 4, **characterised in that** the instrument on the proximal side comprises a handle (44), which is coupled in movement to the actuation rod (38).

6. An endoscopic instrument according to one of the preceding claims, **characterised in that** at least one channel is formed within the hollow shank (2) and this channel runs out in the region of the distal hollow shank end.

7. An endoscopic instrument according to one of the preceding claims, **characterised in that** the drive shaft (14) forms a channel for supplying and/or leading away rinsing fluid.

8. An endoscopic instrument according to one of the claims 4 to 5, **characterised in that** the actuation rod (38) forms a channel for supplying and/or leading away rinsing fluid.

## Revendications

1. Instrument endoscopique pour l'enlèvement de matière, de tissu et équivalents, comportant une tige creuse (2) rigide et un outil (8) en l'extrémité distale de l'instrument, qui peut être manoeuvré de façon pivotante par l'intermédiaire d'un arbre d'entraînement (14) guidé dans la tige creuse (2), l'outil qui dépasse au-delà de l'extrémité distale de la tige creuse (2) pouvant être plié par rapport à l'extrémité distale de la tige creuse (2), l'outil (8) étant ce faisant monté dans une tête (6) articulée de façon à pouvoir se replier à l'extrémité distale de la tige creuse (2) pour le rangement de l'outil (8), **caractérisé en ce que** l'outil (8) est fixé de façon amovible au moyen d'un élément de verrouillage (10) dans la tête (6).

2. Instrument endoscopique selon la revendication 1, **caractérisé en ce que** l'outil (8) est couplé en mouvement à l'arbre d'entraînement (14), directement ou indirectement, par l'intermédiaire d'une articulation à cardan (18).

3. Instrument endoscopique selon l'une des revendications précédentes, **caractérisé en ce que** l'arbre d'entraînement (14) est conçu en deux parties au moins.

4. Instrument endoscopique selon l'une des revendications précédentes, **caractérisé en ce que** la tête (6) est couplée en mouvement à une tige de commande (38), mobile dans le sens axial dans la tige creuse (2), par l'intermédiaire d'un élément articulé (42).

5. Instrument endoscopique selon la revendication 4, **caractérisé en ce que** l'instrument comporte côté proximal une poignée (44), qui est couplée en mouvement à la tige de commande (38).

6. Instrument endoscopique selon l'une des revendications précédentes, **caractérisé en ce qu'**est formé à l'intérieur de la tige creuse (2) au moins un canal, qui débouche dans la zone de l'extrémité distale de la tige creuse.

7. Instrument endoscopique selon l'une des revendications précédentes, **caractérisé en ce que** l'arbre d'entraînement (14) forme un canal pour introduire et/ou pour évacuer du liquide de lavage.

8. Instrument endoscopique selon l'une des revendications 4 ou 5, **caractérisé en ce que** la tige de commande (38) forme un canal pour introduire et/ou pour évacuer du liquide de lavage.
